# EUROPEAN PATENT APPLICATION

(11) **EP 4 563 565 A1**
(43) Date of publication of application: **04.06.2025**
(21) Application number: 23846599.1
(22) Date of filing: 27.07.2023
(51) Int. Cl.: C07C 17/281, C07B 61/00, C07C 17/20, C07C 17/25, C07C 19/10, C07C 21/04

(54) **METHOD FOR PRODUCING CHLOROFLUOROBUTANE (CFB)**

(30) Priority: 28.07.2022 JP 2022120775
(71) Applicant: Kanto Denka Kogyo Co., Ltd., Tokyo 100-0005 (JP)
(72) Inventor: KIKUCHI, Sho, Kurashiki-shi, Okayama 712-8533 (JP); OKUYAMA, Yohei, Kurashiki-shi, Okayama 712-8533 (JP); KISHI, Kazuki, Kurashiki-shi, Okayama 712-8533 (JP)
(74) Representative: Pfenning, Meinig & Partner mbB
(86) International application number: PCT/JP2023/027479
(87) International publication number: WO 2024/024869

(57) **Abstract**

There is provided a method for producing chlorofluorobutane (CFB) by forming chlorinated butadiene by dimerizing trichloroethylene (TCE) and fluorinating hydrogen atoms of the chlorinated butadiene. Trichloroethylene (TCE) is dimerized by using a dimer represented by the following formula (1) as a solvent and in the presence of a free radical generating agent at a temperature of 100°C or higher. Hexachlorobutadiene (C₄Cl₆) is produced from the above dimer by chlorination and dehydrochlorination reaction, and further, the chlorofluorobutane (CFB) is produced by fluorinating the hexachlorobutadiene.

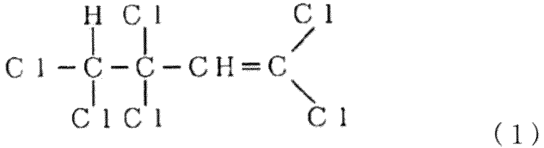

## Description

### TECHNICAL FIELD

The present invention relates to a method for producing chlorofluorobutane (CFB), particularly to a method for producing CFB by forming chlorinated butadiene by dimerizing trichloroethylene (TCE), and fluorinating hydrogen atoms of the chlorinated butadiene.

### BACKGROUND ART

TCE is by-produced in various synthesis reactions, but is a compound whose environmental impact is apprehended. Hence, it is demanded that TCE is, without being accumulated, effectively utilized. It is known that TCE is dimerized in the presence of a free radical generating agent to form chlorinated butylene (PTL 1). Although the method disclosed in PTL 1 is useful for forming a chlorinated butane skeleton or a chlorinated butadiene skeleton from TCE, since being a small-scale method (the amount of TCE used is no more than 5 g), in order to mass produce the dimer, further improvement is needed.

On the other hand, in recent years, etching gases low in the environmental load, such as hexafluorobutadiene (perfluorobutadiene, HFB), have been used, and a mass production method thereof is also proposed (PTL 2). The method for producing HFB by fluorinating chlorinated butane or chlorinated butadiene is studied.

NPL 1 describes a method of producing chlorofluorobutane by carrying out a fluorination reaction by making ClF₃ and ClF to successively act on 1,2,3,4-tetrachloro-1,3-butadiene. NPL 1 also describes that hexafluorobutadiene (perfluorobutadiene, HFB) can be produced by dechlorinating 1,2,3,4-tetrachloro-1,1,2,3,4,4-hexafluorobutane (C₄Cl₄F₆).

### CITATION LIST

### PATENT LITERATURE

PTL 1: Japanese Patent No. 3951331
PTL 2: Japanese Patent No. 5005681

### NON PATENT LITERATURE

NPL 1: J. Muray, J. Chem. Soc., p1884 (1959)

### SUMMARY OF INVENTION

TECHNICAL PROBLEM

A first problem of the present invention is to provide a method for efficiently producing a dimer of TCE. A second problem of the present invention is to provide a series of integrated processes in which chlorinated butane or chlorinated butadiene is produced from TCE, and further fluorinated to produce fluorinated butane or fluorinated butadiene.

### SOLUTION TO PROBLEM

The present invention provides the following.
[1] A method for producing a dimer of trichloroethylene (TCE) represented by the following formula (1): 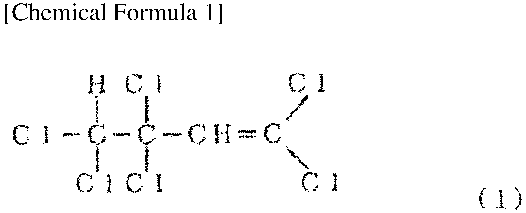 from TCE, the method comprising a step of allowing TCE to dimerize by using a dimer represented by formula (1) as a solvent at a temperature of 100°C or higher in the presence of a free radical generating agent.
[2] The method according to [1], wherein the reaction is carried out at the temperature of 100 to 200°C.
[3] The method according to [1] or [2], wherein the reaction is carried out at atmospheric pressure.
[4] A method for producing hexachlorobutadiene (C₄Cl₆) by chlorination and dehydrochlorination reaction from a dimer of trichloroethylene (TCE) represented by the following formula (1): 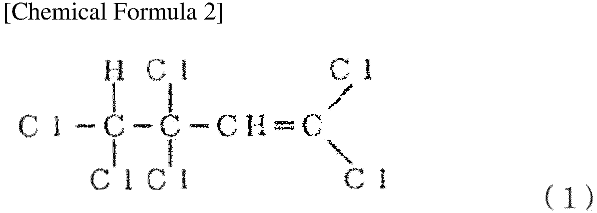 wherein the method comprises a step of reacting the dimer of TCE with chlorine gas (Cl₂) in the presence of a Lewis acid catalyst.
[5] The method according to [4], wherein the reaction is carried out at a temperature of 30 to 200°C.
[6] The method according to [4] or [5], wherein the Lewis acid catalyst contains FeCl₃.
[7] A method for producing 1,2,3,4-tetrachloro-1,1,2,3,4,4-hexafluorobutane from trichloroethylene (TCE), comprising: step (A) of producing a dimer of trichloroethylene (TCE) represented by the following formula (1): 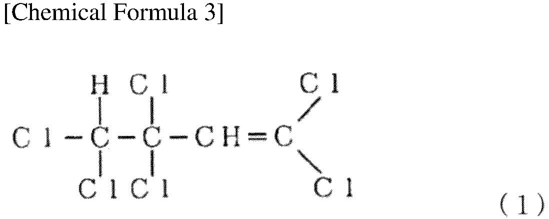
   from TCE, wherein step (A) comprises allowing TCE to dimerize by using a dimer represented by formula (1) as a solvent at a temperature of 100°C or higher in the presence of a free radical generating agent;
   step (B) of producing hexachlorobutadiene (C₄Cl₆) by chlorination and dehydrochlorination reaction from the dimer of TCE represented by the above formula (1), wherein step (B) comprises reacting the dimer of TCE with chlorine gas (Cl₂) in the presence of FeCl₃ as a reaction catalyst;
   step (C) of producing a fluorinated product containing 1, 1,2,3,4,4-hexachloro- 1,2,3,4-tetrafluorobutane by fluorinating the hexachlorobutadiene (C₄Cl₆) with a fluorinating agent; and
   step (D) of producing 1,2,3,4-tetrachloro-1,1,2,3,4,4-hexafluorobutane by reacting the fluorinated product obtained in step (C) with chlorine monofluoride (ClF).
[8] The method according to [7], wherein the fluorinated product obtained in the above step (C) further contains 1,1,1,2,3,4,4-heptachloro-1,2,3-trifluorobutane (C₄Cl₇F₃) and 1,1,2,3,4-pentachloro-1,2,3,4,4-pentafluorobutane (C₄Cl₅F₅).

### ADVANTAGEOUS EFFECTS OF INVENTION

According to the present invention, a dimer of TCE can efficiently be produced. According to the present invention, also chlorinated butane or chlorinated butadiene can efficiently be produced from TCE, which is relatively easily available. Since fluorinated butane or fluorinated butadiene can be produced by further fluorinating the obtained chlorinated butane or chlorinated butadiene, etching gasses low in the environmental load, such as HFB, can be produced inexpensively and massively by a series of integrated processes.

### DESCRIPTION OF EMBODIMENTS

### (Action)

It is known that for the dimerization reaction of trichloroethylene (TCE), making the reaction temperature to be 100°C or higher is effective in order to efficiently progress a free radical reaction (PTL 1). On the other hand, since the boiling point of TCE is 87°C, in order to set the reaction temperature to be 100°C or higher, a pressurized reaction needs to be carried out in a closed pressure vessel. The present inventors have noticed that the boiling point of a dimer of TCE represented by the following formula (1): 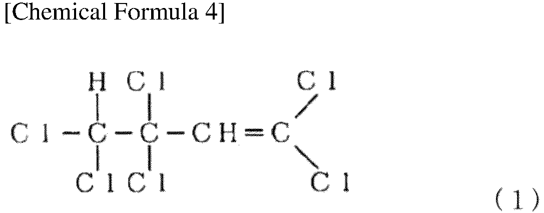 is 205°C (calculated value), and have succeeded in carrying out the reaction at a temperature of 100°C or higher without using a pressure vessel by using the dimer as a reaction solvent. Since a dimer being a product is the reaction solvent, refinement of the product can be carried out easily by removing TCE being a raw material.

For fluorination of the dimer of TCE, a method, not disclosed in NPL 1, was attempted in which fluorine gas (F₂) was made to act, and it has been found that 1,1,2,3,4,4-hexachloro-1,2,3,4-tetrafluorobutane (C₄Cl₆F₄) can efficiently be produced by first making chlorine gas (Cl₂) to act on the dimer of TCE in the presence of a catalyst to form hexachlorobutadiene (HCB), and then making F₂ to act on the HCB. By making ClF to act on the obtained C₄Cl₆F₄, the C₄Cl₆F₄ is converted to 1,2,3,4-tetrachloro-1,1,2,3,4,4-hexafluorobutane (C₄Cl₄F₆), and by dechlorinating the C₄Cl₄F₆, hexafluorobutadiene (perfluorobutadiene, HFB) can be produced.

### (Dimerization reaction of trichloroethylene (TCE))

In the present invention, since the dimerization reaction can be carried out by heating to a temperature of 100°C or higher at the atmospheric pressure, a pressure-resistant vessel is not needed. The reaction temperature is preferably 100 to 200°C and more preferably 120 to 150°C. The reaction product contains unreacted TCE and a dimer of TCE, but TCE can easily be removed by distillation. The reaction product from which TCE has been removed is composed substantially of the dimer of TCE and can be used as it is in the following step.

### (Chlorination and dehydrochlorination reaction of the dimer of TCE)

In the present invention, a step of producing hexachlorobutadiene (C₄Cl₆) from the dimer of TCE by chlorination and dehydrochlorination reaction is carried out, and the step comprises reacting the dimer of TCE with chlorine gas (Cl₂) in the presence of a Lewis acid catalyst. By making hexachlorobutadiene (C₄Cl₆) to be an intermediate, positions to be fluorinated in the following step can be limited and the selectivity of the target substance can be enhanced. The temperatures of the chlorination and the dehydrochlorination reaction of the dimer of TCE are preferably 30 to 200°C, more preferably 50 to 200°C and still more preferably 120 to 150°C. The Lewis acid catalyst includes AlCl₃, SnCl₄, TiCl₄, FeCl₃, ZnCl₂, AlF₃, MgF₂, CaF₂, CrF₃ and ZrF₄, but is most preferably FeCl₃.

### (Fluorination reaction of hexachlorobutadiene (CaCl₆))

In the present invention, a fluorinated product containing 1,1,2,3,4,4-hexachloro-1,2,3,4-tetrafluorobutane is produced by fluorinating hexachlorobutadiene (C₄Cl₆) with a fluorinating agent. In this fluorination reaction, although in addition to the 1,1,2,3,4,4-hexachloro-1,2,3,4-tetrafluorobutane, 1,1,1,2,3,4,4-heptachloro-1,2,3-trifluorobutane (C₄Cl₇F₃) and 1,1,2,3,4-pentachloro-1,2,3,4,4-pentafluorobutane (C₄Cl₅F₅) are produced and contained in the fluorinated product, since any compound can be converted to the target substance 1,2,3,4-tetrachloro-1,1,2,3,4,4-hexafluorobutane (C₄Cl₄F₆) in the following step, there is no need for separation by refinement.

The fluorinating agent is not especially limited, but includes fluorine gas (F₂), chlorine trifluoride (ClF₃) and chlorine monofluoride (CIF). From the viewpoint of the selectivity of 1,1,2,3,4,4-hexachloro-1,2,3,4-tetrafluorobutane, fluorine gas (F₂) is preferable.

### (Reaction of the fluorinated product containing 1,1,2,3,4,4-hexachloro-1,2,3,4-tetrafluorobutane with chlorine monofluoride (CIF))

The reaction can be carried out by bringing CIF into contact with 1,1,2,3,4,4-hexachloro-1,2,3,4-tetrafluorobutane. The reaction temperature is preferably 50 to 200°C and more preferably 80 to 150°C. In this reaction, by-products in the preceding step, 1,1,1,2,3,4,4-heptachloro-1,2,3-trifluorobutane (C₄Cl₇F₃) and 1,1,2,3,4-pentachloro-1,2,3,4,4-pentafluorobutane (C₄Cl₅F₅) are also converted to the target substance 1,2,3,4-tetrachloro-1,1,2,3,4,4-hexafluorobutane (C₄Cl₄F₆). The advantage of adopting this step resides in that since 1,1,1,2,3,4,4-heptachloro-1,2,3-trifluorobutane (C₄Cl₇F₃) and 1,1,2,3,4-pentachloro-1,2,3,4,4-pentafluorobutane (C₄Cl₅F₅) can be converted to tetrachlorohexafluorobutane, no refining step is needed. That is, the reaction condition enhancing the selectivity of the target substance includes introducing CIF at a low flow rate and homogeneously and mildly reacting 1,1,2,3,4,4-hexachloro-1,2,3,4-tetrafluorobutane with ClF. Specifically, the following condition is preferable.

### <Reaction condition>

(a) The ClF flow rate is, for example, in the case where the reaction is carried out in a laboratory scale using 20 to 1,000 g of a raw material liquid, preferably 0.6 to 30 L/hr and more preferably 0.6 to 12 L/hr.
(b) It is preferable that by using an aeration plate of a sintered metallic filter or the like for a bubbling nozzle of ClF, bubbles are micronized. More preferably, a sintered metallic filter of 5 to 500 µm in filter diameter is used.
(c) A usable material of a reactor is an alloy such as stainless steel, Monel metal, inconel or Hastelloy, glass, nickel, polytetrafluoroethylene (PTFE), a perfluoroalkoxy alkane (PFA) or the like. Then, in order to secure the residence time, it is preferable to use a vertical reactor.

### (Dechlorination reaction of 1,2,3,4-tetrachloro-1,1,2,3,4,4-hexafluorobutane)

Hexafluorobutadiene (HFB) can be produced by dechlorinating 1,2,3,4-tetrachloro-1,1,2,3,4,4-hexafluorobutane. Although this reaction is well known in the art, in the present invention, by making novel findings to be reflected in a series of intermediate steps, HFB can be produced inexpensively and massively from TCE.

Examples of the reaction condition include the following.
- The kind of a metal as a reaction catalyst: magnesium, zinc, cadmium, aluminum, copper, sodium, lithium or the like can be used, and the kind is preferably magnesium or zinc.
- The form of the metal as the reaction catalyst: the form is preferably granular or powdery.
- Reaction solvent: an organic solvent or a mixed solvent of an organic solvent and water can be used. Water can also be used singly. The organic solvent is preferably alcohols, and methanol, ethanol, isopropyl alcohol, octanol and the like can be used.
- Reaction temperature: the temperature is preferably 20 to 150°C and more preferably 30 to 90°C.

### EXAMPLES

The present invention will be described specifically by way of the following Examples, but the scope of the present invention is not limited to the following Examples. The following Examples were carried out according to the following reaction scheme.

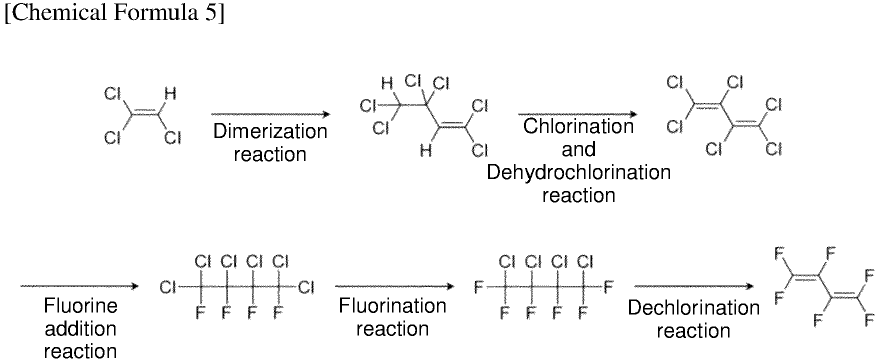

### (Example 1: Dimerization reaction of TCE (2C₂Cl₃H → C₄Cl₆H₂))

In a 300-ml glass-made flask with a dropping funnel and a condenser connected therewith, 75 g of a dimer of TCE (C₄Cl₆H₂) represented by the above formula (1) as a reaction solvent was charged; 25 g of TCE as a raw material was added; 0.5 g of di-tert-butyl peroxide as a free radical generating agent was added; and while the reactor was heated to 140°C and the TCE was refluxed, the reaction was carried out for 20 hours. As a result of analysis of the reaction liquid, a dimer of TCE (C₄Cl₆H₂) was obtained in a yield of 53% from the TCE. The TCE could be separated from the reaction liquid by using an evaporator or the like, enabling the dimer of TCE (C₄Cl₆H₂) as a product to be recovered.

### (Comparative Example 1: Dimerization reaction of TCE (2C₂Cl₃H → C₄Cl₆H₂)

30 g of TCE was charged in a 50 ml-volume fluororesin-made closed vessel; without using a solvent, 0.2 g of di-tert-butyl peroxide as a free radical generating agent was added; and while the reactor was heated to 150°C, the reaction was carried out for 5 hours. As a result of analysis of the reaction liquid, a dimer of TCE (C₄Cl₆H₂) was obtained in a yield of 50%.

Example 1 and Comparative Example 1 were compared and according to the present invention, a dimer of TCE could be obtained in a good yield (53%) without using a closed pressure vessel. The dimer of TCE could easily be refined by removing the unreacted raw material by distillation. It has been found that since in the present invention, there was no need for using a pressure vessel, the present invention was suitable as one step in a continuous integrated processes.

### (Example 2: Chlorination and dehydrochlorination reaction (C₄Cl₆H₂ → C₄Cl₆)

In a 500-ml glass-made flask with a condenser connected therewith, 390 g of a dimer of TCE (C₄Cl₆H₂) was charged; 0.4 g of FeCl₃ as a reaction catalyst was added; the reactor was heated to 90°C and chlorine gas (Cl₂) was bubbled at 6 L/hr; and after 8 hours, the reaction was terminated. Thereafter, the reactor heating temperature was changed to 150°C and the heating was continued for 4 hours. Nitrogen gas was blown in from a gas introduction port to purge chlorine gas dissolved in the reaction liquid, and thereafter, the reactor was opened and the reaction liquid was taken out. As a result of analysis by GC of the reaction liquid, hexachlorobutadiene (C₄Cl₆) was obtained in a yield of 93%.

### (Example 3: Fluorine addition reaction with fluorine gas (F₂))

In a SUS-made reactor equipped with a gas introduction port, a gas outlet port and a thermometer, 450 g of hexachlorobutadiene (C₄Cl₆) was added; and a fluorine gas (F₂) of 20% by volume with respect to the total of nitrogen gas and fluorine gas was bubbled at a flow rate of 24 L/hr for 17 hours under ice cooling. Then, nitrogen was bubbled at the same flow rate for 1 hour to thereby obtain 544 g of a reaction liquid. As a result of analysis by NMR of the reaction liquid, 1,1,2,3,4,4-hexachloro-1,2,3,4-tetrafluorobutane formed by fluorine addition to unsaturated bonds of hexachlorobutadiene was contained in a composition ratio of 54% by mass. Further, intermediates, which were capable of serving as raw materials of a target substance, 1,2,3,4-tetrachloro-1,1,2,3,4,4-hexafluorobutane, in a later step (Example 4), C₄Cl₇F₃ (1,1,1,2,3,4,4-heptachloro-1,2,3-trifluorobutane) and C₄Cl₅F₅ (1,1,2,3,4-pentachloro-1,2,3,4,4-pentafluorobutane) were contained in composition ratios of 12% by mass and 7% by mass, respectively.

### (Example 4: Fluorination reaction with chlorine monofluoride (ClF))

200 g of the reaction liquid by the fluorine addition reaction containing 1,1,2,3,4,4-hexachloro-1,2,3,4-tetrafluorobutane, obtained in Example 3, was added to a SUS-made reactor equipped with a gas introduction port, a gas outlet port and a thermometer. On the gas output port side, an ice cooling trap and a -20°C condenser were installed. The reactor was heated to 120°C and chlorine monofluoride was bubbled at a flow rate of 2.4 L/hr for 16 hours. Then, nitrogen was bubbled at the same flow rate for 1 hour to thereby obtain 161 g of a reaction liquid. As a result of analysis of the reaction liquid, 1,2,3,4-tetrachloro-1,1,2,3,4,4-hexafluorobutane was contained in a composition ratio of 56% by mass.

### (Example 5: Fluorine addition reaction with chlorine trifluoride (ClF₃))

In a SUS-made reactor equipped with a gas introduction port, a gas outlet port and a thermometer, 345 g of hexachlorobutadiene (C₄Cl₆) was added; and a chlorine trifluoride gas of 50% by volume with respect to the total concentration of nitrogen and chlorine trifluoride was bubbled at a flow rate of 4.8 L/hr for 21 hours under ice cooling. Then, nitrogen was bubbled at a flow rate of 12 L/hr for 1 hour to thereby obtain 460 g of a reaction liquid. As a result of analysis by NMR of the reaction liquid, 1,1,2,3,4,4-hexachloro-1,2,3,4-tetrafluorobutane formed by fluorine addition to unsaturated bonds of hexachlorobutadiene was contained in a composition ratio of 31% by mass. Further, intermediates, which were capable of serving as raw materials of a target substance, 1,2,3,4-tetrachloro-1,1,2,3,4,4-hexafluorobutane, in a later step (Example 4), C₄Cl₇F₃ (1,1,1,2,3,4,4-heptachloro-1,2,3-trifluorobutane) and C₄Cl₅F₅ (1,1,2,3,4-pentachloro-1,2,3,4,4-pentafluorobutane) were contained in composition ratios of 17% by mass and 3% by mass, respectively.

Example 3 and Example 5 were compared and it has been found that the F₂ gas gave a higher selectivity of 1,1,2,3,4,4-hexachloro-1,2,3,4-tetrafluorobutane than the ClF₃ gas. Either of the products obtained in Example 3 and Example 5 could be converted to a target substance, 1,2,3,4-tetrachloro-1,1,2,3,4,4-hexafluorobutane (C₄Cl₄F₆), in a later step (Example 4), and the C₄Cl₄F₆ could be converted to hexafluorobutadiene (HFB) by being subjected to dechlorination reaction by a known method (Example 6). According to the present invention, since chlorinated butane or chlorinated butadiene could be produced efficiently from TCE, which was relatively easily available, and further, fluorinated butane or fluorinated butadiene could be produced, an etching gas low in the environmental load, such as HFB, could be produced inexpensively and massively.

### (Example 6: Dechlorination reaction with a metal)

To a 300-ml glass-made reactor, a water cooling condenser and a dropping funnel were connected, and to the tip of the cooling condenser, a 500-ml SUS-made gas collecting vessel with a valve was connected. 70 g of a solvent, isopropyl alcohol, and 41 g of a granular metal zinc were charged in the reactor; and the reactor was heated to 70°C under stirring and the gas collecting vessel was cooled in a dry ice-acetone bath. Then, a distillate containing 40 g of 1,2,3,4-tetrachloro-1,1,2,3,4,4-hexafluorobutane obtained by distilling the reaction liquid of Example 4 was dropwise charged in the reaction liquid over 1 hour by using a dropping funnel, and thereafter allowed to react for 5 hours. As a result of analysis of a product collected in the gas collecting vessel after the finish of the reaction, the HFB purity was 97 GC%. Then, the yield thereof based on 1,2,3,4-tetrachloro-1,1,2,3,4,4-hexafluorobutane was 94% by mol.

## Claims

1. A method for producing a dimer of trichloroethylene (TCE) represented by the following formula (1): 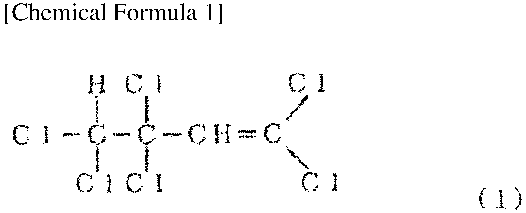 from TCE, the method comprising a step of allowing TCE to dimerize by using a dimer represented by formula (1) as a solvent at a temperature of 100°C or higher in the presence of a free radical generating agent.

2. The method according to claim 1, wherein the reaction is carried out at the temperature of 100 to 200°C.

3. The method according to claim 1 or 2, wherein the reaction is carried out at atmospheric pressure.

4. A method for producing hexachlorobutadiene (C₄Cl₆) by chlorination and dehydrochlorination reaction from a dimer of trichloroethylene (TCE) represented by the following formula (1): 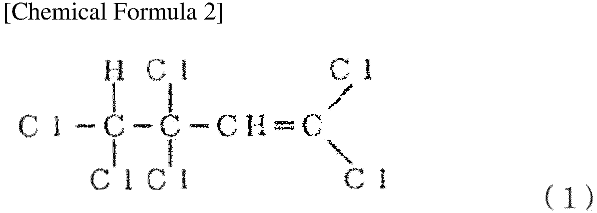 wherein the method comprises a step of reacting the dimer of TCE with chlorine gas (Cl₂) in the presence of a Lewis acid catalyst.

5. The method according to claim 4, wherein the reaction is carried out at a temperature of 30 to 200°C.

6. The method according to claim 4 or 5, wherein the Lewis acid catalyst comprises FeCl₃.

7. A method for producing 1,2,3,4-tetrachloro-1,1,2,3,4,4-hexafluorobutane from trichloroethylene (TCE), comprising:
step (A) of producing a dimer of trichloroethylene (TCE) represented by the following formula (1): 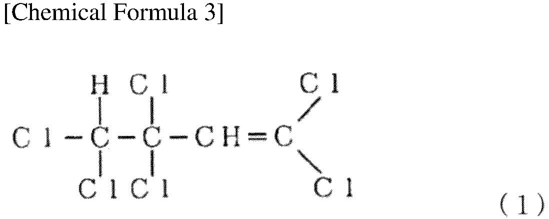 from TCE, wherein step (A) comprises allowing TCE to dimerize by using a dimer represented by formula (1) as a solvent at a temperature of 100°C or higher in the presence of a free radical generating agent;
step (B) of producing hexachlorobutadiene (C₄Cl₆) by chlorination and dehydrochlorination reaction from the dimer of TCE represented by the above formula (1), wherein step (B) comprises reacting the dimer of TCE with chlorine gas (Cl₂) in the presence of FeCl₃ as a reaction catalyst;
step (C) of producing a fluorinated product comprising 1,1,2,3,4,4-hexachloro-1,2,3,4-tetrafluorobutane by fluorinating the hexachlorobutadiene (C₄Cl₆) with a fluorinating agent; and
step (D) of producing 1,2,3,4-tetrachloro-1,1,2,3,4,4-hexafluorobutane by reacting the fluorinated product obtained in step (C) with chlorine monofluoride (ClF).

8. The method according to claim 7, wherein the fluorinated product obtained in the above step (C) further comprises 1,1,1,2,3,4,4-heptachloro-1,2,3-trifluorobutane (C₄Cl₇F₃) and 1,1,2,3,4-pentachloro-1,2,3,4,4-pentafluorobutane (C₄Cl₅F₅).
